(19) Europäisches Patentamt
European Patent Office
Office européen des brevets

(11) EP 3 974 528 A1

(12) **EUROPEAN PATENT APPLICATION**
published in accordance with Art. 153(4) EPC

(43) Date of publication:
**30.03.2022 Bulletin 2022/13**

(21) Application number: **20810164.2**

(22) Date of filing: **19.05.2020**

(51) International Patent Classification (IPC):
***C12N 15/11** (2006.01)* ***C12Q 1/68** (2018.01)*

(52) Cooperative Patent Classification (CPC):
**C12N 15/11; C12Q 1/68**

(86) International application number:
**PCT/JP2020/019790**

(87) International publication number:
**WO 2020/235563 (26.11.2020 Gazette 2020/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.05.2019 JP 2019094216**

(71) Applicant: **Kyushu University, National University Corporation**
**Nishi-ku**
**Fukuoka-shi**
**Fukuoka 819-0395 (JP)**

(72) Inventors:
- **OKI Shinya**
  **Fukuoka-shi, Fukuoka 819-0395 (JP)**
- **OHKAWA Yasuyuki**
  **Fukuoka-shi, Fukuoka 819-0395 (JP)**

(74) Representative: **Epping - Hermann - Fischer Patentanwaltsgesellschaft mbH**
**Schloßschmidstraße 5**
**80639 München (DE)**

# (54) OLIGONUCLEOTIDE, OMICS ANALYSIS METHOD, AND OMICS ANALYSIS KIT

(57) An oligonucleotide includes an additional sequence for detecting a nucleic acid. At least one nucleotide in the additional sequence is modified with a photolabile protecting group.

FIG. 2A

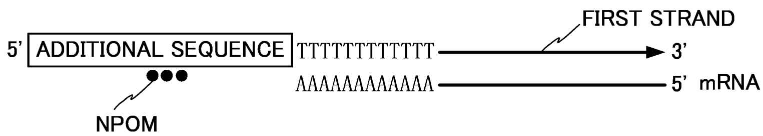


FIG. 2B

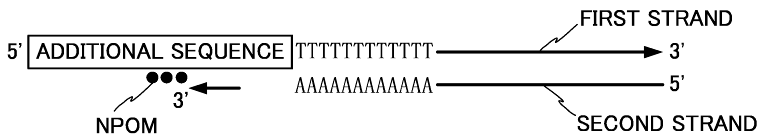


FIG. 2C

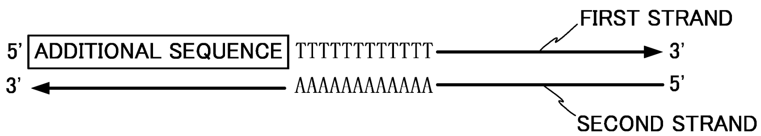


EP 3 974 528 A1

SEQUENCE LISTING

<110> Kyushu University, National University Corporation

<120> Oligonucleotide, method of omics analysis and kit for omics analysis

<130> 20F028-PCT

<150> JP 2019-94216
<151> 2019-05-20

<160> 20

<170> PatentIn version 3.5

<210> 1
<211> 82
<212> DNA
<213> Artificial Sequence

<220>
<223> OligoDNA B

<220>
<221> misc_feature
<222> (46)..(51)
<223> n is a, c, g, or t

<400> 1
gccggtaata cgactcacta tagggagttc tacagtccga cgatcnnnnn ntgcagattt 60

tttttttttt tttttttttt tv 82

<210> 2
<211> 82
<212> DNA
<213> Artificial Sequence

<220>
<223> OligoDNA C

<220>
<221> misc_feature
<222> (46)..(51)
<223> n is a, c, g, or t

<400> 2
gccggtaata cgactcacta tagggagttc tacagtccga cgatcnnnnn ncaactcttt 60

tttttttttt tttttttttt tv 82

<210> 3
<211> 86
<212> DNA
<213> Artificial Sequence

<220>
<223> OligoDNA D

<220>
<221> misc_feature
<222> (50)..(55)
<223> n is a, c, g, or t

<400> 3
gccggtaata cgactcacta tagggtttga gttctacagt ccgacgatcn nnnnnagact 60

cttttttttt tttttttttt tttttv 86

<210> 4
<211> 86
<212> DNA
<213> Artificial Sequence

<220>
<223> OligoDNA F

<220>
<221> misc_feature
<222> (50)..(55)
<223> n is a, c, g, or t

<400> 4
gccggtaata cgactcacta tagggtttga gttctacagt ccgacgatcn nnnnnagcta 60

gttttttttt tttttttttt tttttv 86

<210> 5
<211> 86
<212> DNA
<213> Artificial Sequence

<220>
<223> OligoDNA G

<220>
<221> misc_feature
<222> (50)..(55)
<223> n is a, c, g, or t

<400> 5
gccggtaata cgactcacta tagggtttga gttctacagt ccgacgatcn nnnnntcgaa 60

gttttttttt tttttttttt tttttv 86

<210> 6
<211> 86
<212> DNA
<213> Artificial Sequence

<220>
<223> OligoDNA H

<220>

<221> misc_feature
<222> (50)..(55)
<223> n is a, c, g, or t

<400> 6
gccggtaata cgactcacta tagggtttga gttctacagt ccgacgatcn nnnnngtctc 60

attttttttt tttttttttt tttttv 86

<210> 7
<211> 28
<212> DNA
<213> Artificial Sequence

<220>
<223> Random oligoDNA

<220>
<221> misc_feature
<222> (23)..(28)
<223> n is a, c, g, or t

<400> 7
gccttggcac ccgagaattc cannnnnn 28

<210> 8
<211> 32
<212> DNA
<213> Artificial Sequence

<220>
<223> Forward primer for Gapdh

<400> 8
cgacaggttc agagttctac agtccgacga tc 32

<210> 9
<211> 31
<212> DNA
<213> Artificial Sequence

<220>
<223> Reverse primer for Gapdh

<400> 9
acaatttcca tcccagaccc ccataataac c 31

<210> 10
<211> 24
<212> DNA
<213> Artificial Sequence

<220>
<223> Forward primer for Sox2

<400> 10
gccggtaata cgactcacta tagg 24

<210> 11
<211> 26
<212> DNA
<213> Artificial Sequence

<220>
<223> Reverse primer for Sox2

<400> 11
gattcggctc tgttattgga atcagg 26

<210> 12
<211> 35
<212> DNA
<213> Artificial Sequence

<220>
<223> Probe for Sox2

<400> 12
tctcaaactg tgcataatgg agtaaaaact taagt 35

<210> 13
<211> 24
<212> DNA
<213> Artificial Sequence

<220>
<223> Forward primer for Gapdh

<400> 13
gccggtaata cgactcacta tagg 24

<210> 14
<211> 20
<212> DNA
<213> Artificial Sequence

<220>
<223> Reverse primer for Gapdh

<400> 14
cagcaaggac actgagcaag 20

<210> 15
<211> 23
<212> DNA
<213> Artificial Sequence

<220>
<223> Probe for Gapdh

<400> 15
gtgggtgcag cgaactttat tga 23

<210> 16
<211> 36

<212> DNA
<213> Artificial Sequence

<220>
<223> OligoDNA I

<400> 16
tcgtcggcag cgtctttaga tgtgtataag agacag 36

<210> 17
<211> 19
<212> DNA
<213> Artificial Sequence

<220>
<223> OligoDNA J

<400> 17
ctgtctctta tacacatct 19

<210> 18
<211> 37
<212> DNA
<213> Artificial Sequence

<220>
<223> OligoDNA K

<400> 18
gtctcgtggg ctcggtttag atgtgtataa gagacag 37

<210> 19
<211> 61
<212> DNA
<213> Artificial Sequence

<220>
<223> Forward primer

<220>
<221> misc_feature
<222> (30)..(37)
<223> n is a, c, g, or t

<400> 19
aatgatacgg cgaccaccga gatctacacn nnnnnnntcg tcggcagcgt ctttagatgt 60

g 61

<210> 20
<211> 56
<212> DNA
<213> Artificial Sequence

<220>
<223> Reverse primer

```
<220>
<221>  misc_feature
<222>  (25)..(32)
<223>  n is a, c, g, or t

<400>  20
caagcagaag acggcatacg agatnnnnnn nngtctcgtg ggctcggttt agatgt              56
```

## Description

Technical Field

The present disclosure relates to an oligonucleotide, an omics analysis method, and an omics analysis kit.

Background Art

Methods of acquiring omics information, such as RNA sequence analysis (RNA-seq), comprehensive open chromatin region analysis (ATAC-seq) and genome methylated region analysis (Bisulfite-seq), using next-generation sequencers capable of determining a large number of base sequences have been established.

Non Patent Literature 1 discloses cell expression by linear amplification and sequencing (CEL-Seq) using linear amplification with *in vitro* transcription (IVT) reaction, as an example of RNA-seq specialized in a small number of cells.

Non Patent Literature 2 discloses CEL-Seq2 obtained by improving CEL-Seq to have improved sensitivity. In CEL-Seq2, first, the poly A terminal of an mRNA in a sample is hybridized with an oligonucleotide including a PolyT sequence and an additional sequence including a T7 promoter sequence at the 5' end, and a first strand is synthesized by a reverse transcriptase. Subsequently, a second strand that is the complementary strand of the first strand is synthesized. Then, IVT reaction is allowed to proceed by T7 RNA polymerase. As a result, a base sequence closer to the 3' end than the T7 promoter sequence can be amplified as an RNA. A library is synthesized by reverse transcription reaction and polymerase chain reaction (PCR), and the base sequence thereof is analyzed with a sequencer.

Citation List

Non Patent Literature

Non Patent Literature 1: Tamar Hashimshony and three others, "CEL-Seq: Single-Cell RNA-Seq by Multiplexed Linear Amplification", Cell Rep., 2012, 2(3), 666-673

Non Patent Literature 2: Tamar Hashimshony and twelve others, "CEL-Seq2: sensitive highly-multiplexed single-cell RNA-Seq", Genome Biology, 2016, 17(77)


Summary of Invention

Technical Problem

In accordance with CEL-Seq and CEL-Seq2, it is necessary to isolate a cell although gene expression information in each cell can be acquired. For isolating such a cell, it is necessary to fractionate the cell by sorting of the cell, cutout of the cell by laser microdissection, or the like.

Such fractionation of a cell as described above may result in damage to the cell. Therefore, omics information obtained from the fractionated cell may differ from the actual state of the living body under the influence of the damage. Moreover, there is a problem that spatial resolution is restricted depending on a fractionation method.

The present disclosure is made under such actual circumstances with an objective to provide an oligonucleotide, an omics analysis method, and an omics analysis kit, in which omics information further reflecting the actual state of a living body can be acquired under high spatial resolution.

Solution to Problem

An oligonucleotide according to a first aspect of the present disclosure includes:

an additional sequence for detecting a nucleic acid,
wherein at least one nucleotide in the additional sequence is modified with a photolabile protecting group.

In such a case, it is also acceptable that the photolabile protecting group is 6-nitropiperonyloxymethyl group bonded to a base of the nucleotide.

It is also acceptable that the oligonucleotide according to the first aspect of the present disclosure further includes at least one nucleotide modified with a photolabile protecting group at a 3' end of the additional sequence.

It is also acceptable that the additional sequence includes a discrimination sequence for discrimination.

It is also acceptable that the additional sequence includes a promoter sequence that is a base sequence involved in initiation of transcription; and
the nucleotide is located at a position closer to a 3' end than the promoter sequence.
It is also acceptable that the oligonucleotide according to the first aspect of the present disclosure further includes a transposon sequence at a position closer to a 3' end than the additional sequence.
An omics analysis method according to a second aspect of the present disclosure includes:

an exposure step of exposing the oligonucleotide according to the first aspect of the present disclosure to a tissue section or a cell mass;
a first synthesis step of elongating the oligonucleotide with an mRNA, included in the tissue section or the cell mass, as a template to synthesize a first strand;
an irradiation step of irradiating a part of the tissue section or the cell mass with light;
a second synthesis step of synthesizing a second strand complementary to the first strand with the first strand as a template; and
a determination step of determining a base sequence of the first strand and the second strand.

An omics analysis method according to a third aspect of the present disclosure includes:

an exposure step of exposing the oligonucleotide according to the first aspect of the present disclosure to a tissue section or a cell mass;
a first synthesis step of elongating the oligonucleotide with a DNA, included in the tissue section or the cell mass, as a template to synthesize a first strand;
an irradiation step of irradiating a part of the tissue section or the cell mass with light;
a second synthesis step of synthesizing a second strand complementary to the first strand with the first strand as a template; and
a determination step of determining a base sequence of the first strand and the second strand.

An omics analysis method according to a fourth aspect of the present disclosure includes:

an exposure step of exposing the oligonucleotide according to the first aspect of the present disclosure and a transposase to a tissue section or a cell mass;
an irradiation step of irradiating a part of the tissue section or the cell mass with light;
a reaction step of performing fill-in of a 5' protruding end of a DNA fragment collected from the tissue section or the cell mass; and
a determination step of determining a base sequence of the DNA fragment subjected to the fill-in.

An omics analysis kit according to a fifth aspect of the present disclosure includes:
the oligonucleotide according to the first aspect of the present disclosure.

Advantageous Effects of Invention

In accordance with the present disclosure, omics information further reflecting the actual state of a living body can be acquired under high spatial resolution.

Brief Description of Drawings

FIG. 1 is a view illustrating the base sequences of oligonucleotides according to an embodiment of the present disclosure, and the positions of photolabile protecting groups in the base sequences;
FIG. 2 is a view illustrating conceptual diagrams for explaining CEL-Seq2 using the oligonucleotides according to the present embodiment of the present disclosure, in which FIG. 2A illustrates the elongation reaction of a first strand, FIG. 2B illustrates an aspect in which irradiation with light is not performed before the synthesis reaction of a second strand, and FIG. 2C illustrates an aspect in which the irradiation with light is performed before the synthesis reaction of the second strand;
FIG. 3 is a view illustrating conceptual diagrams for explaining a method of analyzing a genomic sequence using an oligonucleotide according to another embodiment of the present disclosure, in which FIG. 3A illustrates the structure of the oligonucleotide, FIG. 3B illustrates the oligonucleotide inserted into a genomic DNA, and FIG. 3C illustrates a DNA fragment obtained from a DNA fragment in a region irradiated with light, and subjected to fill-in;

FIG. 4 is a view illustrating the base sequences of oligodeoxyribonucleotides (DNAs) according to Example 1, and the positions of photolabile protecting groups in the base sequences;

FIG. 5 is a view indicating the results of the quantification of Gapdh genes in quantitative PCR (qPCR) for cDNAs obtained in CEL-seq2 of cultured cells using the oligodeoxyribonucleotides illustrated in FIG. 4;

FIG. 6 is a view illustrating the results of electrophoresis of libraries constructed using the oligodeoxyribonucleotides illustrated in FIG. 4;

FIG. 7 is a view illustrating the expression of a Sox2 gene in a tissue section according to Example 2, in which FIG. 7A illustrates a position at which irradiation with ultraviolet rays (UV) is performed in the tissue section, and FIG. 7B illustrates the results of immunostaining according to the Sox2 gene;

FIG. 8 is a view indicating the results of qPCR according to the Sox2 gene and a Gapdh gene in Example 2, in which each of FIG. 8A and FIG. 8B indicates the expression levels of the Sox2 gene and the Gapdh gene;

FIG. 9 is a view illustrating the base sequences of oligoDNAs according to Example 3, and the positions of photolabile protecting groups in the base sequences; and

FIG. 10 is a view illustrating the results of the electrophoresis of reaction products according to Example 3.

## Description of Embodiments

Embodiments according to the present disclosure will be described with reference to the drawings. The present disclosure is not limited to the following embodiments.

### Embodiment 1

Each of oligonucleotides according to the present embodiment includes an additional sequence for detecting a nucleic acid. The additional sequence is a base sequence designed to detect a nucleic acid. Preferably, the additional sequence is a base sequence that does not hybridize with a target nucleic acid in a living body to be detected. For example, the additional sequence is a base sequence added to at least one of the 5' end or the 3' end of a nucleic acid in a living body to be detected or a nucleic acid complementary to the nucleic acid.

More specific examples of the additional sequence include an adapter sequence that is a consensus sequence for amplifying or detecting a nucleic acid, a promoter sequence involved in initiation of transcription, and a discrimination sequence for discrimination. The adapter sequence is, for example, a primer binding sequence for PCR, or a base sequence for performing amplification by bridge PCR in a next-generation sequencer or the like. The promoter sequence is, for example, a T7, T3, or SP6 promoter sequence used in IVT or the like in order to detect a nucleic acid.

Examples of the discrimination sequence include a bar code sequence and a unique molecular identifier (UMI) sequence. The bar code sequence is an artificial sequence determined in advance for each sample to be analyzed. When each of the oligonucleotides is used in a plurality of samples, bar code sequences with different base sequences are assigned to the samples, respectively. The UMI sequence is a random base sequence. The discrimination sequence may include both or either of the bar code sequence and the UMI sequence. When used in CEL-Seq2, the additional sequence preferably includes the T7 promoter sequence, the adapter sequence, the UMI sequence, and the bar code sequence from the 5' end to the 3' end.

In each of the oligonucleotides according to the present embodiment, at least one nucleotide in the additional sequence is modified with a photolabile protecting group. The photolabile protecting group is bound to a base of the nucleotide in the base sequence of each of the oligonucleotides. The protecting group is not particularly limited as long as formation of a hydrogen bond between the base and a base complementary to the base is inhibited, and the protecting group is decomposed by irradiation with light to be able to result in the cancellation of the inhibition of the formation of the hydrogen bond. For example, the protecting group is 2-(2-nitrophenyl)propyl group, 2-(2-nitrophenyl)propyloxymethyl group, 1-(2-nitrophenyl)ethyl group, 6-nitropiperonyloxymethyl group (NPOM), or the like. Preferably, the photolabile protecting group is NPOM. The base into which the photolabile protecting group is introduced is preferably thymine (T). However, the base is not limited to thymine, and may be adenine (A), cytosine (C), or guanine (G) depending on the protecting group used.

FIG. 1 illustrates the base sequences of the oligonucleotides 1 to 6 according to the present embodiment used in CEL-Seq2, and the positions of the photolabile protecting groups. In each of the oligonucleotides 1 to 6, the base sequence enclosed with the dashed line is an additional sequence. Each of the additional sequences included in the oligonucleotides 1 to 6 includes a T7 promoter sequence, an adapter sequence, a UMI sequence, and a bar code sequence. In FIG. 1, "t", to which "●" is attached, is deoxythymidine modified with a photolabile protecting group. As illustrated in FIG. 1, the photolabile protecting groups are located in the additional sequences. Each oligonucleotide may include at least one nucleotide modified with such a photolabile protecting group, and preferably includes a plurality of such nucleotides.

It is preferable that when the additional sequence includes a promoter sequence such as a T7 promoter, a nucleotide modified with a photolabile protecting group is located at a position closer to the 3' end than the promoter sequence, for example, between the promoter sequence and an adapter sequence, as in the case of the oligonucleotides 3 to 6.

Such an oligonucleotide may further include at least one nucleotide modified with a photolabile protecting group at the 3' end of an additional sequence, as well as the photolabile protecting groups located in the additional sequence. For example, like the oligonucleotides 2, and 4 to 6 illustrated in FIG. 1, such an oligonucleotide according to the present embodiment includes nucleotides modified with one to three photolabile protecting groups at the 3' end of the additional sequence.

Such an oligonucleotide according to the present embodiment has a sequence length of, for example, 10 to 120 bases, 20 to 110 bases, 50 to 100 bases, 60 to 95 bases, or 70 to 90 bases. When used in CEL-Seq2, the oligonucleotide preferably has a sequence length of 80 to 90 bases. The oligonucleotide according to the present embodiment can be synthesized by a known method. Such an oligonucleotide is also referred to as "polynucleotide".

When such an oligonucleotide according to the present embodiment is used as a template for the elongation reaction of a nucleic acid, the formation of a hydrogen bond between a base modified with a photolabile protecting group and a base complementary to the base is inhibited. Therefore, in the elongation reaction, a region complementary to a base sequence from a nucleotide modified with a photolabile protecting group to the 5' end in the oligonucleotide is not elongated in a nucleic acid to be elongated. When the oligonucleotide is irradiated with light, the photolabile protecting group is decomposed, the region complementary to the base sequence from the nucleotide modified with the photolabile protecting group to the 5' end in the oligonucleotide is elongated.

For example, when such an oligonucleotide according to the present embodiment is used in CEL-Seq2, the oligonucleotide hybridizing with an mRNA through polyT is elongated by a reverse transcriptase, to obtain a first strand, as illustrated in FIG. 2A. When a second strand is elongated with the first strand as a template, an elongation reaction stops at a position with respect to a nucleotide modified with a photolabile protecting group in an additional sequence, as illustrated in FIG. 2B, and a region between the position and the 3' end, that is, a part of the additional sequence lacks the second strand. Only when the oligonucleotide is irradiated with light to decompose the photolabile protecting group, the inhibition of the elongation reaction by the photolabile protecting group is canceled to obtain the second strand including an additional sequence having a full length, as illustrated in FIG. 2C.

In accordance with such an oligonucleotide according to the present embodiment, a nucleic acid including an optional additional sequence can be obtained only when the oligonucleotide is irradiated with light. Since the nucleic acid can be detected through the additional sequence, for example, it is possible to irradiate a part of a tissue section or a cell mass with light, and to thereby detect a nucleic acid present in the tissue section or the cell mass, only in the part irradiated with the light. Thus, the oligonucleotide is useful in an omics analysis method in which omics information according to a predetermined region irradiated with light is acquired. The omics analysis method using the oligonucleotide will be described below by taking a method of analyzing gene expression as an example.

When such an oligonucleotide according to the present embodiment is used in the method of analyzing gene expression, the oligonucleotide is preferably applied to CEL-Seq2. The method of analyzing gene expression includes an exposure step, a first synthesis step, a second synthesis step, and a determination step. The method of analyzing gene expression using the oligonucleotide described above will be described below by taking CEL-Seq2 as an example. The oligonucleotide includes an adapter sequence and a T7 promoter sequence at a position closer to the 5' end side than the adapter sequence in an additional sequence.

In the exposure step, the oligonucleotide described above is exposed to a tissue section or a cell mass. The tissue section is an optional tissue from an animal or a plant. The tissue section is prepared by a known method such as a paraffin method or a freezing method. To enhance the penetrability of the tissue section, the tissue section may be subjected to known treatment. The thickness of the section is 3 to 15 $\mu$m, 4 to 12 $\mu$m, or 5 to 10 $\mu$m. The size of the section is not limited, but is, for example, not less than 1 mm $\times$ 1 mm in consideration of the easiness of operation. The cell mass is an optional cell population from an animal or a plant. To expose the oligonucleotide to the tissue section or the cell mass, a reagent containing the oligonucleotide may be dropwise added to the tissue section or the cell mass. Preferably, the oligonucleotide, together with the reagent used for elongating the oligonucleotide in the first synthesis step, is dropwise added to the tissue section or the cell mass.

In the first synthesis step, the oligonucleotide is elongated with an mRNA, included in the tissue section or the cell mass, as a template, to synthesize a first strand. The elongation reaction of the oligonucleotide with the mRNA as the template may be performed by a known method. For example, a synthetic reagent for synthesizing the first strand is exposed to the tissue section or the cell mass in the first synthesis step. The synthetic reagent includes dNTP and a reverse transcriptase, and may further include an RNase inhibitor. Reaction conditions in the synthesis of the first strand are set as appropriate depending on the synthetic reagent used.

In an irradiation step, a part of the tissue section or the cell mass is irradiated with light. Depending on the size of a region to be analyzed in the tissue section, a part of the tissue section may be irradiated with light under a microscope.

In the case of using the microscope, the magnification of a visual field is, for example, 2 to 500 times, 10 to 300 times, 20 to 200 times, or 50 to 150 times. The wavelength of the light is adjusted depending on the protecting group included in the oligonucleotide described above. When the protecting group is NPOM, the light is UV, and the wavelength of the UV is preferably 340 to 380 nm. Light irradiation time is set depending on the protecting group and the wavelength of the light. When the protecting group is NPOM, the light irradiation time is, for example, 10 to 30 minutes, 12 to 20 minutes, or 13 to 18 minutes. The tissue section or the cell mass may be washed with phosphate buffered saline (PBS) and/or the like before the part of the tissue section or the cell mass is irradiated with the light.

In the second synthesis step, a second strand complementary to the first strand is synthesized with the first strand as a template. For example, in the second synthesis step, the synthesis of the complementary strand may be performed by a known method with all cDNAs, included in the tissue section lysed using Proteinase K and/or the like, as templates. A synthetic reagent for synthesizing the second strand includes, for example, dNTP, RNaseH, DNA ligase, and DNA polymerase. Reaction conditions in the synthesis of the second strand are set as appropriate depending on the synthetic reagent used.

In the determination step, the base sequence of the first strand or the second strand is determined. In the determination step, a double strand DNA including the first and second strands obtained in the second synthesis step is purified, and IVT reaction is performed, whereby a sequence downstream of the T7 promoter is transcribed to synthesize a cRNA. The purification of the double strand cDNA and the IVT reaction can be performed by a known method, a commercially available kit, and/or the like.

Subsequently, a cDNA is synthesized from the cRNA, synthesized in the IVT, using a reverse transcriptase. A library is obtained by amplifying the cDNA by PCR, and purifying the resultant. The cDNA included in the library includes an additional sequence at an end. Thus, sequence analysis and the like can be performed through the additional sequence.

Particularly when the additional sequence includes a bar code sequence as a discrimination sequence, a plurality of samples can be discriminated through the bar code sequence even in the case of allowing the samples to coexist. When the additional sequence includes a UMI sequence as a discrimination sequence, an amplified product that is a product amplified in duplicate in library amplification in PCR, IVT, or the like can be determined through the UMI sequence.

In such an oligonucleotide according to the present embodiment, a photolabile protecting group in an additional sequence is decomposed only in a region, irradiated with light, of a tissue section or the like, to synthesize a nucleic acid including a complete additional sequence designed in advance, as described in detail above. The target nucleic acid in the region, irradiated with light, of the tissue section or the like can be detected through the additional sequence by using an elongation reaction such as PCR. Since a limited region of 1 $\mu m^2$ or less in a tissue or a cell mass can be irradiated with light such as UV, the light enables the omics information of the tissue or the cell mass to be acquired with high spatial resolution. As a result, the need for fractionating a cell by sorting of a cell, cutout of a cell mass by laser microdissection, or the like is eliminated, and therefore, omics information further reflecting the actual state of a living body can be acquired without damaging the cell.

It has been described that such an oligonucleotide according to the present embodiment may include nucleotides modified with one to three photolabile protecting groups at the 3' end of an additional sequence. However, it is also acceptable that the additional sequence includes a polyT sequence at the 3' end, and one to three deoxythymidines at the 5' end of the polyT sequence are modified with photolabile protecting groups. A portion closer to the 3' end of the oligonucleotide according to the present embodiment may be a random sequence or a sequence complementary to a part of an mRNA or a genomic DNA, as well as the polyT sequence, and a deoxythymidine included in the sequence may be modified with a photolabile protecting group.

Moreover, it has been described that in the oligonucleotide according to the present embodiment, the additional sequence may include a discrimination sequence. As a result, even when a plurality of samples is allowed to coexist, the discrimination of the samples, and the like can be performed by associating the plurality of samples with discrimination sequences with different base sequences, particularly bar code sequences, respectively. Moreover, libraries duplicated in library amplification in PCR, IVT reaction, or the like can be determined by allowing the discrimination sequences to include UMI sequences.

In another embodiment, a method of analyzing a genomic sequence is provided as an omics analysis method. The method of analyzing a genomic sequence includes a first synthesis step different from the first synthesis step in the above-described method of analyzing gene expression, and the other steps similar to the other steps in the method of analyzing gene expression. In the first synthesis step in the method of analyzing a genomic sequence, the oligonucleotide is elongated with a DNA, included in a tissue section or a cell mass, as a template, to synthesize a first strand. In the method of analyzing a genomic sequence, a double strand DNA including first and second strands obtained in a second synthesis step may be subjected to PCR, to construct a library. Since the DNA included in the library includes an additional sequence at an end, the base sequences of the first and second strands can be determined by performing sequence analysis and/or the like through the additional sequence.

When the DNA included in the tissue section or the cell mass has been subjected to bisulfite treatment by a known method before the first synthesis step in the method of analyzing a genomic sequence, genome methylation information can also be acquired by the method of analyzing a genomic sequence. The oligonucleotide according to the present embodiment, used in the method of analyzing a genomic sequence, need not include a promoter sequence in an additional sequence.

In another embodiment, the oligonucleotide described above can be applied to a method of analyzing a genomic sequence, in which a transposase is utilized. The method of analyzing a genomic sequence, in which the transposase is utilized, includes an exposure step, an irradiation step, a reaction step, and a determination step. With regard to each step of the method of analyzing a genomic sequence, in which the transposase is utilized, points different from those of the above-described method of analyzing gene expression with the mRNA as a template or the above-described method of analyzing a genomic sequence with a genomic DNA as a template will be mainly described below.

As illustrated in FIG. 3A, the oligonucleotide used in the method of analyzing a genomic sequence, in which the transposase is utilized, further includes transposon sequences at positions closer to the 3' end than additional sequences X and Y including nucleotides modified with NPOM (denoted by "●"). The additional sequences may include a T7 promoter sequence, an adapter sequence, a UMI sequence, a bar code sequence, and/or the like. Such a transposon sequence is a base sequence of which the position on a genome can be transferred in a cell by the action of a transposase. The transposon sequence is allowed to be a recognition sequence for a Tn5 transposase. Mixing of a double strand oligonucleotide solution and a Tn5 transposase, illustrated in FIG. 3A, causes the double strand oligonucleotide and the Tn5 transposase to form a complex.

In the exposure step, the above-described complex (that is, the oligonucleotide according to the present embodiment and the transposase) is exposed to a tissue section or a cell mass. Since the oligonucleotide includes the transposon sequences, the exposure of the complex to the genomic DNA results in insertion (tagmentation) of such oligonucleotides into the various regions of a genomic DNA, as illustrated in FIG. 3B. As a result, the additional sequences X and Y, together with the transposon sequences, are inserted into the various genome regions.

After the irradiation step, the 5' protruding end of a DNA fragment collected from the tissue section or the cell mass is subjected to fill-in. The fill-in reaction is established only when a protecting group is cleaved by irradiation with light. As a result, the 5' protruding end is subjected to the fill-in only in the DNA fragment obtained from the region irradiated with light in the tissue section or the like, to add the additional sequence X or Y having a full length to the end, as illustrated in FIG. 3C.

In the determination step, the base sequence of the DNA fragment subjected to the fill-in is determined. Since the DNA fragment subjected to the fill-in includes the additional sequence X or Y with a double strand at the end, a nucleic acid such as a genomic DNA can be amplified by PCR using a primer for the additional sequence X or Y even if the amount of the nucleic acid is minute. In such a manner, various genome regions can be amplified and analyzed.

Exposure of the complex to a cell nucleus in an unfixed state results in selective insertion of the oligonucleotide into an open chromatin region. Then, the end of the collected DNA fragment is subjected to fill-in through the irradiation step, the DNA fragment is amplified by PCR reaction, and the base sequence of the DNA fragment is determined, whereby the open chromatin region can be identified (ATAC-seq).

The transposon sequence is not particularly limited. However, a known transposon sequence such as a Tn5 transposon, a Tn7 transposon, and a Tn9 transposon can be used as the transposon sequence. A transposase corresponding to the transposon sequence may be used as the transposase.

Such an oligonucleotide according to the present embodiment can be used in various technologies in which a target nucleic acid can be detected through an additional sequence. Examples of such technologies include qPCR. It is acceptable that qPCR is a probe method or an intercalater method. The oligonucleotide according to the present embodiment can also be applied to a fluorescence *in situ* hybridization (FISH) method.

## Embodiment 2

An omics analysis kit according to the present embodiment may include such an oligonucleotide described in Embodiment 1 as described above. Preferably, the omics analysis kit further includes a reagent for elongating an oligonucleotide. In the case of the above-described CEL-Seq2 and method of analyzing a genomic sequence, the reagent for elongating an oligonucleotide with a nucleic acid as a template is a reagent for synthesizing a first strand. In the case of the method of analyzing a genomic sequence, in which a transposase is utilized, the reagent includes the transposase to be inserted into the region, and a reagent for the fill-in and PCR amplification of the DNA fragment after the insertion. Specific examples of the reagent include a transposase such as Tn5, DNA polymerase, dNTP, and a buffer. The reagent may further include a reducing agent such as dithiothreitol (DTT).

The omics analysis kit may further include a reagent for synthesizing a second strand. Examples of the reagent for synthesizing the second strand include dNTP, RNaseH, DNA ligase, and DNA polymerase. Preferably, the omics analysis kit according to the present embodiment includes a plurality of kinds of oligonucleotides, and the oligonucleotides include different discrimination sequences in additional sequences, respectively. More specifically, the plurality of the kinds of the oligonucleotides includes, as the discrimination sequences in the additional sequences, bar code sequences including different base sequences, respectively. For example, the omics analysis kit includes: a first oligonucleotide including a first bar code sequence as a discrimination sequence in an additional sequence; and a second oligonucleotide including a second bar code sequence, of which the base sequence is different from the base sequence of the first bar code sequence, as a discrimination sequence in an additional sequence. The number of the kinds of the oligonucleotides including the different bar code sequences, respectively, is not particularly limited, and may be two or more.

The omics analysis kit according to the present embodiment is suitable in the omics analysis method in which the oligonucleotides according to Embodiment 1 as described above are used. The omics analysis kit includes a reagent used in the omics analysis method, and therefore enables omics information further reflecting the actual state of a living body to be efficiently acquired with high spatial resolution.

In the omics analysis kit according to the present embodiment, the additional sequences may include the discrimination sequences, and the oligonucleotides may be the plurality of the kinds of oligonucleotides in which the discrimination sequences are different from each other. Even when libraries derived from a plurality of samples are allowed to coexist depending on the discrimination sequences, it is possible to discriminate or reconstruct sample information using a next-generation sequencer. Moreover, placement of UMI sequences in the additional sequences enables determination of libraries duplicated in library amplification in PCR, IVT reaction, or the like.

## Examples

The present disclosure will be more specifically described with reference to Examples as described below. However, the present disclosure is not limited to Examples.

### Example 1

(Synthesis of OligoDNA)

OligoDNAs B to H illustrated in FIG. 4 were synthesized using NPOM Caged-dT-CE Phosphoramidite (5'-dimethoxytrityl-N3-[[1-(6-nitro-1,3-benzodioxol-5-yl)ethoxy]methyl]-2'-deoxythymidine,3'-[(2-cyanoethyl)-(N,N-diisopropyl)]-phosphoramidite; produced by Glen Research, Catalog number: 10-1534-95). The base sequences of oligoDNAs B to D and oligoDNAs F to H are represented by SEQ ID NOS: 1 to 3 and SEQ ID NOS:4 to 6, respectively. The base sequence of the oligoDNA E is the same as the base sequence of the oligoDNA D. In FIG. 4, deoxythymidine modified with NPOM is represented by "t" to which "●" is attached.

(CEL-seq2 of Cultured Cell)

The total RNA of an NIH3T3 cell was purified using RNeasy Mini Kit (manufactured by QIAGEN), and prepared in 10 ng/μL. A sample was prepared by adding 0.5 μL of any (500 ng/μL) of oligoDNAs A to H and 0.5 μL of dNTP Mix (10 mM, manufactured by New England Biolabs (NEB)) to 5μL of the resulting total RNA. To decompose the higher-order structure of the RNA, the sample was heated at 65°C for 5 minutes, and then rapidly cooled. A reverse transcriptase solution was added to the sample. The composition of the reverse transcriptase solution is as follows.

0.5 μL of RNase OUT (40 U/μL, manufactured by Invitrogen)
0.5 μL of Superscript II Reverse Transcriptase (200 U/μL, manufactured by Invitrogen)
5× First Strand Buffer (2μL) attached to the enzyme, and DTT (1 μL)

Reaction was performed at 42°C for 60 minutes, and then at 70°C for 10 minutes, to synthesize a first strand. The sample was irradiated with UV for 15 minutes by the following instruments.

Inverted microscope (Eclipse Ti-U with 10-fold objective lens, manufactured by NIKON CORPORATION)
130 W Mercury lamp apparatus (INTENSILIGHTC-HGFI, manufactured by NIKON CORPORATION)
Fluorescence filter cube (DAPI-5060C, manufactured by Semrock; excitation wavelength of 352 to 402 nm)

After the UV irradiation, a reagent for synthesizing a second strand was added. The composition of the reagent for synthesizing the second strand is as follows.

7 μL of Ultrapure water
2.31 μL of 5× Second strand buffer (manufactured by Invitrogen)

0.23 μL of dNTP Mix (10 mM, manufactured by NEB)
0.08 μL of *E. coli* DNA ligase (10 U/μL; manufactured by Invitrogen)
0.3 μL of *E. coli* DNA Polymerase I (10 U/μL; manufactured by BioLabs)
0.08 μL of *E. coli* RnaseH (2 U/μL, manufactured by Invitrogen)

The second strand was synthesized at 16°C for 2 hours. A DNA was purified with Agencourt AMPure XP (manufactured by Beckman Coulter), and eluted with 6.4 μL of ultrapure water. Each of the following reagents attached to MEGAscript T7 Transcription Kit (manufactured by Ambion) was added in an amount of 1.6 μL.

10 × T7 Reaction buffer

A/G/C/UTP Solution

T7 Enzyme Mix

IVT reaction was performed at 37°C for 17 hours. To remove excess nucleotide and primer, 6 μL of ExoSAP-IT Express PCR Cleanup Reagents (manufactured by Applied Biosystems) was added, and incubation was performed at 37°C for 15 minutes.

To shorten a cRNA synthesized in IVT, a fragmentation buffer (200 mM Tris-acetate, pH 8.1, 500 mM KOAc, 150 mM MgOAc) was added, and the resultant was heated at 94°C for 3 minutes. The resultant was purified with Agencourt RNAClean XP (manufactured by Beckman Coulter), and eluted with 7 μL of ultrapure water, to obtain a sample. The following substances were added to the sample.

1 μL ofRandomhexRT oligodeoxynucleotide (5'-GCCTTGGCACCCGAGAATTCCANNNNNN-3' (SEQ ID NO:7); 250 ng/μL)
0.5 μL of dNTP Mix (10 mM)

To decompose the higher-order structure of the cRNA, the sample was heated at 65°C for 5 minutes, and then rapidly cooled. A reverse transcriptase solution identical with the above was added to the sample. The synthesis of a cDNA was performed at 25°C for 10 minutes, and then at 42°C for 60 minutes. The following PCR reagents and the like were added to the resultant.

9.9 μL of Ultrapure water
22.5 μL of Phusion High-Fidelity PCR Master Mix (manufactured by NEB)
0.23 μL of dNTP Mix (10 mM, manufactured by NEB)
1.8 μL of RNA PCR Primer [RP1] (manufactured by Illumina)
1.8 μL of RNA PCR Primer Index [RPIX] (manufactured by Illumina)

PCR reaction was performed up to 11 cycles, RNase A (10 mg/mL, manufactured by NACALAI TESQUE, INC.) was added to a PCR product, and the resultant was incubated at 37°C for 30 minutes. A DNA was purified with Agencourt AMPure XP, and eluted with 25 μL of ultrapure water. The resultant was electrophoresed on E-Gel precast agarose gel to collect a 250-300 bp fraction. Then, the DNA was purified in QIAGEN MinElute PCR Purification kit to construct a library.

The library constructed, as discussed above, from the sample obtained by adding the oligoDNA D or the oligoDNA G was electrophoresed.

Moreover, qPCR (SYBR Green method) of a Gapdh gene was performed with the reaction product of the cDNA synthesis as a template. The base sequences of forward and reverse primers used in qPCR are represented in SEQ ID NOS:8 and 9, respectively.

(Results)

In the oligoDNAs B to H, a great decrease in signal was observed in UV unirradiation while the expression of a Gapdh gene was detected in the case of UV irradiation, as illustrated in FIG. 5. Particularly in each of the oligoDNAs F to H including at least one deoxythymidine modified with NPOM at the 3' end of the additional sequence, the expression of the Gapdh gene was hardly detected in the UV unirradiation. Signals detected in the UV unirradiation in the oligoDNAs B to E were higher than those in the oligoDNAs F to G. However, since there are differences in the detected signals between the UV irradiation and the UV unirradiation even in the oligoDNAs B to E, irradiation of the wider region of a tissue section with UV is considered to allows the expression of the Gapdh gene in a UV unirradiation region to be hardly detected.

When a library for sequencing was synthesized using the oligoDNA G, the library was detected only in the case of the irradiation with UV while the library was not detected in the UV unirradiation, as illustrated in FIG. 6.

Example 2

(CEL-seq2 of Tissue Section)

A 14-day-old mouse embryo collected from a pregnant mouse was embedded in OCT compound (manufactured by Sakura Finetek). The mouse embryo was immersed in isopentane in which dry ice was put, and frozen rapidly. A tissue section of the mouse embryo, having a thickness of 10 μm, was generated in a cryostat, and air-dried. The tissue section was washed with PBS, and fixed in a 3.7% formalin solution for 10 minutes. To enhance the penetrability of the tissue section, the following treatment was performed.

The tissue section was immersed in 5% Triton/PBS for 3 minutes, and immersed in 0.1 N HCl for 5 minutes. To decompose the higher-order structure of the RNA, the tissue section was neutralized in 1 M Tris-HCl (pH 8.0), immersed in PBS at 65°C for 5 minutes, and cooled rapidly. A reagent for synthesizing a first strand was dropwise added to the tissue section. The composition of the reagent for synthesizing a first strand is as follows.

0.5μL of OligoDNA G (500 ng/μL)
0.5 μL of dNTP Mix (10 mM)
5 μL of Ultrapure water
0.5 μL of RNase OUT
0.5 μL of Superscript II Reverse Transcriptase
5× First Strand Buffer (2 μL)attached to the enzyme, and DTT (1 μL)

The reaction was performed at 42°C for 60 minutes, and then at 70°C for 10 minutes, to synthesize a first strand. The tissue section was washed with PBS. A circular portion having a diameter of about 1.2 mm in each of the neural tube and posterior limb of the tissue section illustrated in FIG. 7A was irradiated with UV for 15 minutes by the following instruments.

Upright microscope (manufactured by Leica Camera AG, DM5000B, 20-fold objective lens)
120 W mercury lamp apparatus (EL6000)
Fluorescence filter cube (manufactured by Leica Camera AG, excitation wavelength of 340 to 380 nm)

The tissue section was immunostained for Sox2. In primary antibody reaction in the immunostaining, a rabbit-derived anti-Sox2 antibody (clone D9B8N, manufactured by Cell Signaling Technology, Inc.) was dropwise added to the tissue section, to cause the reaction for 1 hour. In secondary antibody reaction, Alexa-488-labeled anti-rabbit IgG antibody (manufactured by Invitrogen) was dropwise added to the tissue section, to cause the reaction for 1 hour. In nuclear staining, TO-PRO-3 (manufactured by Invitrogen) was dropwise added to the tissue section, to cause reaction for 10 minutes.

To the tissue section, 80 μL of Proteinase K (400 μg/mL, manufactured by NACALAI TESQUE, INC.) was dropwise added to lyse the tissue section at 55°C for 90 minutes.

(Quantitative PCR)

The qPCR (TaqMan (trade mark) probe method) of Sox2 gene and Gapdh gene was performed with the lysate of the whole tissue section, obtained with Proteinase K, as a template. The base sequences of a forward primer, a reverse primer, and a probe according to the Sox2 gene are represented by SEQ ID NOS:10, 11, and 12, respectively. The base sequences of a forward primer, a reverse primer, and a probe according to the Gapdh gene are represented by SEQ ID NOS: 13, 14, and 15, respectively.

(Results)

The results of the immunostaining are illustrated in FIG. 7B. The Sox2 gene is expressed mainly in the neural tube, but is not expressed in the posterior limb. The results of qPCR according to the Sox2 gene and the Gapdh gene are indicated in FIGS. 8A and 8B, respectively. In FIGS. 8A and 8B, one circle indicates the signal of one tissue section. Neither the Sox2 gene nor the Gapdh gene was detected when the tissue section was not irradiated with UV. In contrast, both the Sox2 gene and the Gapdh gene were detected when the neural tube was irradiated with UV, while only the Gapdh gene was detected when the posterior limb was irradiated with UV Thus, it was revealed that it is possible to analyze gene expression only in a region irradiated with UV

Example 3

(Formation of OligoDNA-Tn5 Complex)

Like Example 1, oligoDNAs I to M illustrated in FIG. 9 were synthesized using NPOM Caged-dT-CE Phosphoramidite. The base sequences of the oligoDNAs I, J, and K are represented by SEQ ID NOS:16, 17, and 18, respectively. The base sequences of the oligoDNAs L and M are the same as the base sequences of the oligoDNAs I and the K, respectively. However, some deoxythymidines in the base sequences of the oligoDNAs L and M are modified with NPOM. In FIG. 9, the deoxythymidines modified with NPOM in the oligoDNAs L and M are represented by "t" to which "●" is attached.

The oligoDNA J and the oligoDNA I, K, L, or M were diluted with an NTE buffer (20 mM Tris-HCl (pH 7.5), 20 mM NaCl and 1 mM ethylenediaminetetraacetic acid (EDTA)) to have a final concentration of 100 μM, and the resultant was heated at 98°C for 15 minutes, and then annealed until reaching 4°C while gently decreasing the temperature of the resultant at a rate of -1°C/min. The sample including the annealed oligoDNAs was mixed with the following reagent, and the mixture was incubated at 23°C for 1 hour to obtain an oligoDNA-Tn5 complex.

0.625 μL of Annealed oligoDNAs
10 μL of 100% Glycerol
5.375 μL of 2× Dialysis buffer (100 mM HEPES-KOH (pH 7.2), 0.2 M NaCl, 0.2 mM EDTA, 2 mM dithiothreitol, 0.2% Triton X-100, and 20% glycerol)
9 μL of Tn5 (11.56 μM)

(Pretreatment of Tissue Section)

Like Example 2, the frozen section of a 14-day-old mouse embryo was generated, and fixed in a 3.7% formalin solution for 10 minutes. The section was immersed in 1% Triton/PBS for 10 minutes, and subjected to permeabilization treatment. Blocking was performed in a solution including Blocking OneP (manufactured by NACALAI TESQUE, INC.) and TBST (Tris-buffered saline and 0.1% Tween20), of which the amounts were equal to each other, for 10 minutes. An obtained tissue section was washed with PBS.

(Insertion of Transposon)

Mixing of 45 μL of 1× TAPS-DMF buffer (10 mM TAPS-NaOH (pH 8.5), 5 mM $MgCl_2$, 10% N,N-dimethylformamide) with 5 μL of the oligoDNA-Tn5 complex prepared as described above, and the mixture was dropwise added to the tissue section prepared as described above. The tissue section was left to stand at 37°C for 1 hour. The tissue section was washed with 0.2% sodium dodecyl sulfate (SDS) for 10 minutes, and washed with PBS.

(UV Irradiation)

A circular portion having a diameter of about 5 mm in the tissue section was irradiated with UV using the following instruments.

Upright microscope (manufactured by Leica Camera AG, DM2500, 5-fold objective lens)
103 W Mercury lamp apparatus (manufactured by Leica Camera AG, ebq 100)
A Fluorescence filter cube (manufactured by Leica Camera AG, excitation wavelength of 340 to 380nm)

(Extraction and Purification of DNA)

To the tissue section, 80 μL of Proteinase K (400 μg/mL, manufactured by NACALAI TESQUE, INC.) was dropwise added, and the tissue section was lysed at 55°C for 90 minutes. The resultant was purified in QIAGEN MinElute PCR Purification kit, and eluted with 22 μL of ultrapure water.

(Fill-in and PCR Amplification)

The following PCR mixture was prepared.

1.5 μL of the Eluate
0.375 μL of Forward primer (SEQ ID NO:19)
0.375 μL of Reverse primer (SEQ ID NO:20)
3.75 μL of NEBnext 2× PCR mix (manufactured by NEB)
1.35 μL of Ultrapure water

It is to be noted that "n" in the base sequences represented by SEQ ID NOS:19 and 20 is an index sequence defined by Illumina.

Fill-in was performed in the following step 1, and amplification reaction (steps 3 to 5 in 25 or 31 cycles) was performed after a step 2.

Step 1: 72°C, 5 minutes
Step 2: 95°C, 30 seconds
Step 3: 95°C, 10 seconds
Step 4: 63°C, 30 seconds
Step 5: 72°C, 60 seconds

A reaction product was electrophoresed on an agarose gel, and stained with SYBR Gold (manufactured by Invitrogen).

(Results)

FIG. 10 illustrates the results of the electrophoresis. When the irradiation with UV was not performed, a genomic DNA was not amplified because there was NPOM (lanes 5 and 6). In contrast, when the irradiation with UV was performed, NPOM was decomposed, and the amplification of the genomic DNA was observed (lanes 7 and 8).

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2019-094216, filed on May 20, 2019, the entire disclosure of which is incorporated by reference herein.

Industrial Applicability

The present disclosure is preferable for omics analysis requiring high spatial resolution.

## Claims

1. An oligonucleotide comprising:

   an additional sequence for detecting a nucleic acid,
   wherein at least one nucleotide in the additional sequence is modified with a photolabile protecting group.

2. The oligonucleotide according to claim 1, wherein the photolabile protecting group is 6-nitropiperonyloxymethyl group bonded to a base of the nucleotide.

3. The oligonucleotide according to claim 1 or 2, further comprising:
   at least one nucleotide modified with a photolabile protecting group at a 3' end of the additional sequence.

4. The oligonucleotide according to any one of claims 1 to 3, wherein the additional sequence comprises a discrimination sequence for discrimination.

5. The oligonucleotide according to any one of claims 1 to 4, wherein the additional sequence comprises a promoter sequence that is a base sequence involved in initiation of transcription, and
   the nucleotide is located at a position closer to a 3' end than the promoter sequence.

6. The oligonucleotide according to any one of claims 1 to 4, further comprising:
   a transposon sequence at a position closer to a 3' end than the additional sequence.

7. An omics analysis method comprising:

   an exposure step of exposing the oligonucleotide according to any one of claims 1 to 5 to a tissue section or a cell mass;
   a first synthesis step of elongating the oligonucleotide with an mRNA, included in the tissue section or the cell mass, as a template to synthesize a first strand;
   an irradiation step of irradiating a part of the tissue section or the cell mass with light;
   a second synthesis step of synthesizing a second strand complementary to the first strand with the first strand as a template; and
   a determination step of determining a base sequence of the first strand and the second strand.

8. An omics analysis method comprising:

   an exposure step of exposing the oligonucleotide according to any one of claims 1 to 4 to a tissue section or a cell mass;
   a first synthesis step of elongating the oligonucleotide with a DNA, included in the tissue section or the cell mass, as a template to synthesize a first strand;
   an irradiation step of irradiating a part of the tissue section or the cell mass with light;
   a second synthesis step of synthesizing a second strand complementary to the first strand with the first strand as a template; and
   a determination step of determining a base sequence of the first strand and the second strand.

9. An omics analysis method comprising:

   an exposure step of exposing the oligonucleotide according to claim 6 and a transposase to a tissue section or a cell mass;

an irradiation step of irradiating a part of the tissue section or the cell mass with light;
a reaction step of performing fill-in of a 5' protruding end of a DNA fragment collected from the tissue section or the cell mass; and
a determination step of determining a base sequence of the DNA fragment subjected to the fill-in.

**10.** An omics analysis kit comprising:
the oligonucleotide according to any one of claims 1 to 6.

FIG. 1

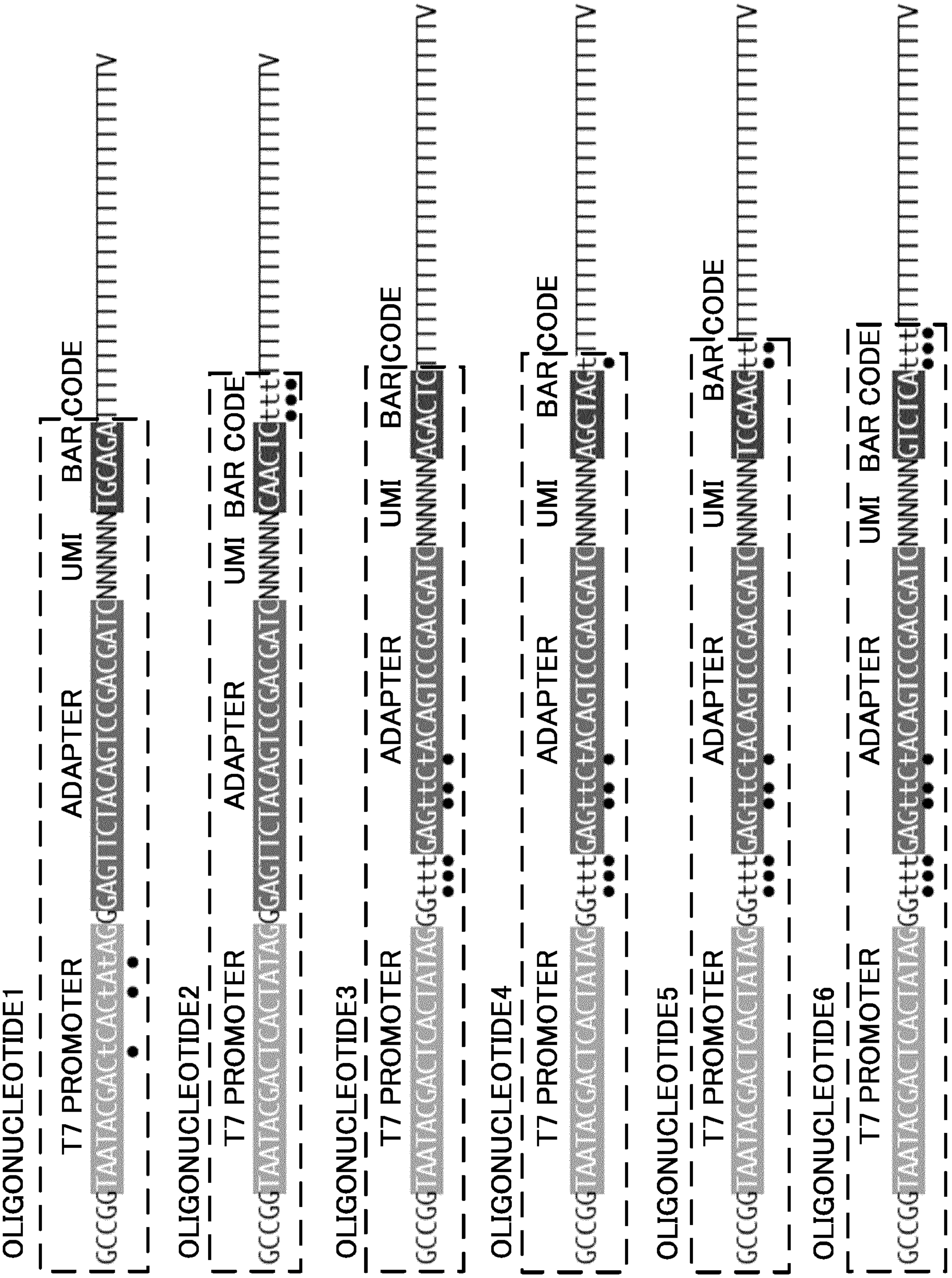
OLIGONUCLEOTIDE1
T7 PROMOTER
ADAPTER
UMI
BAR CODE
OLIGONUCLEOTIDE2
OLIGONUCLEOTIDE3
OLIGONUCLEOTIDE4
OLIGONUCLEOTIDE5
OLIGONUCLEOTIDE6

FIG. 2A

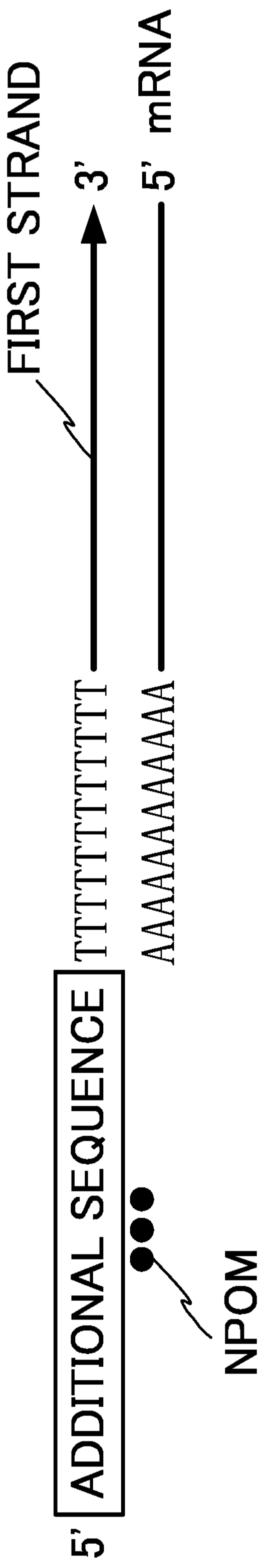


FIG. 2B

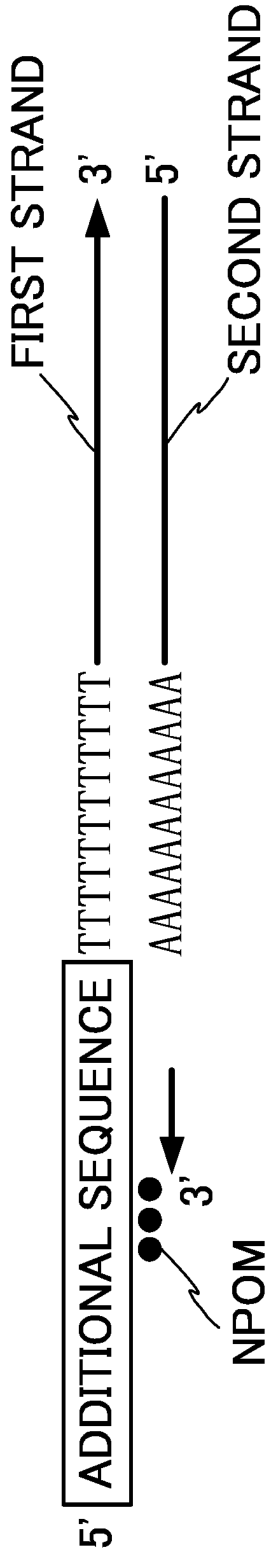


FIG. 2C

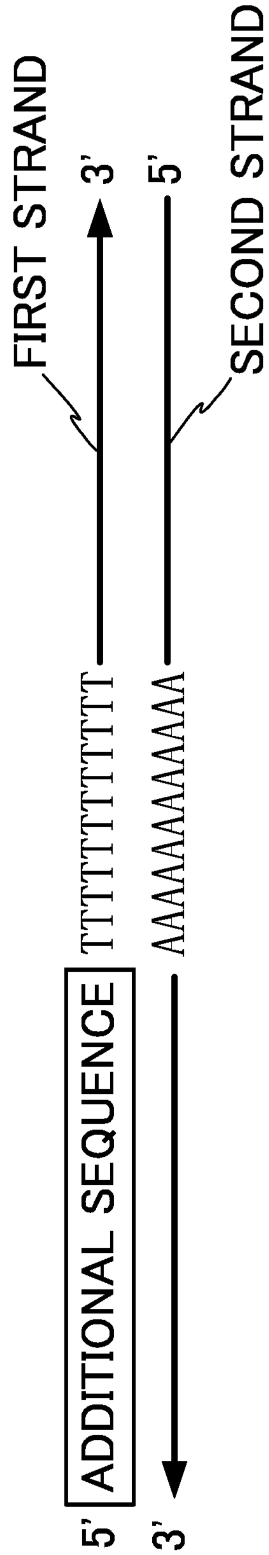

FIG. 3A

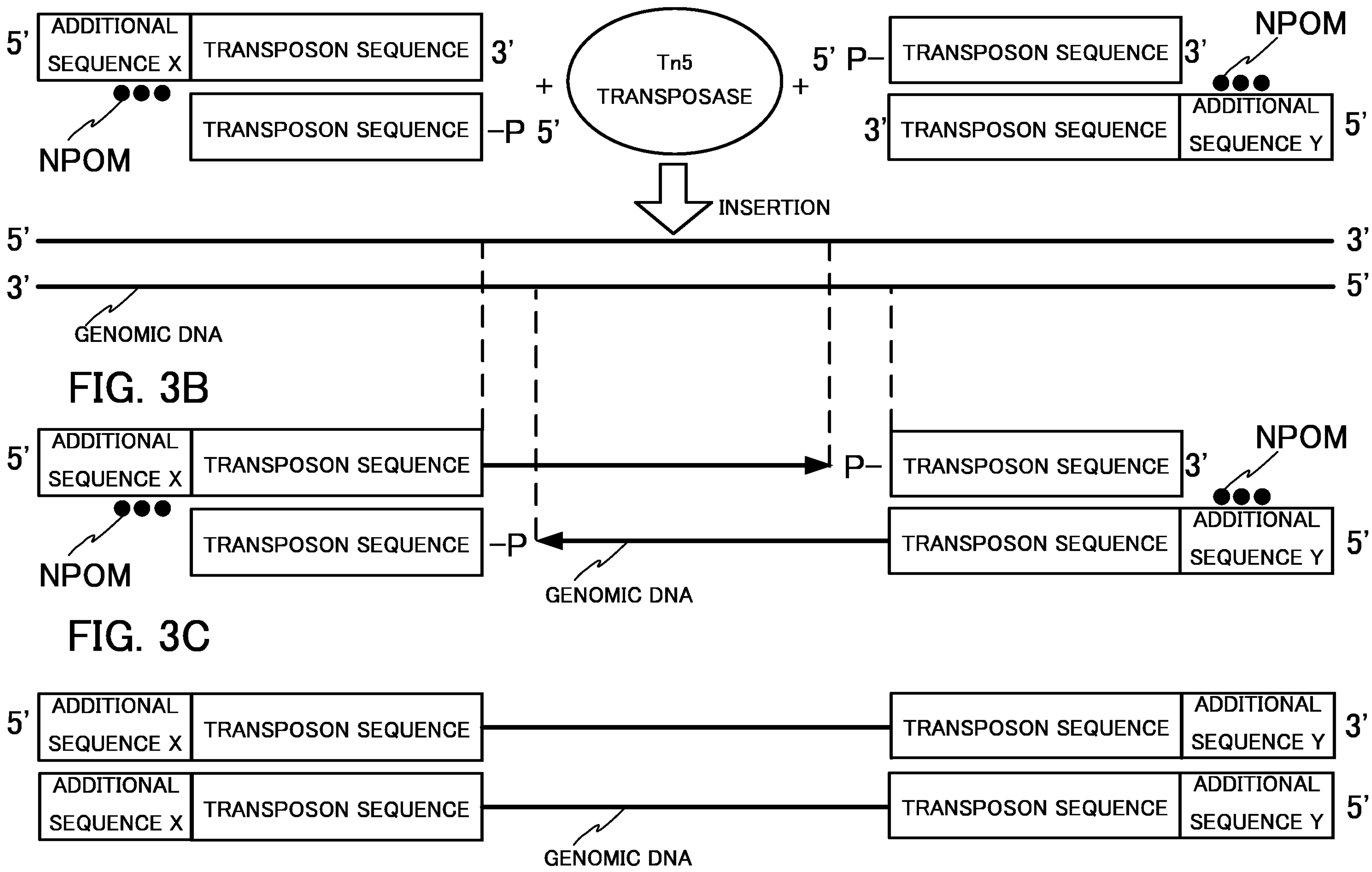


FIG. 3B

FIG. 3C

# FIG. 4

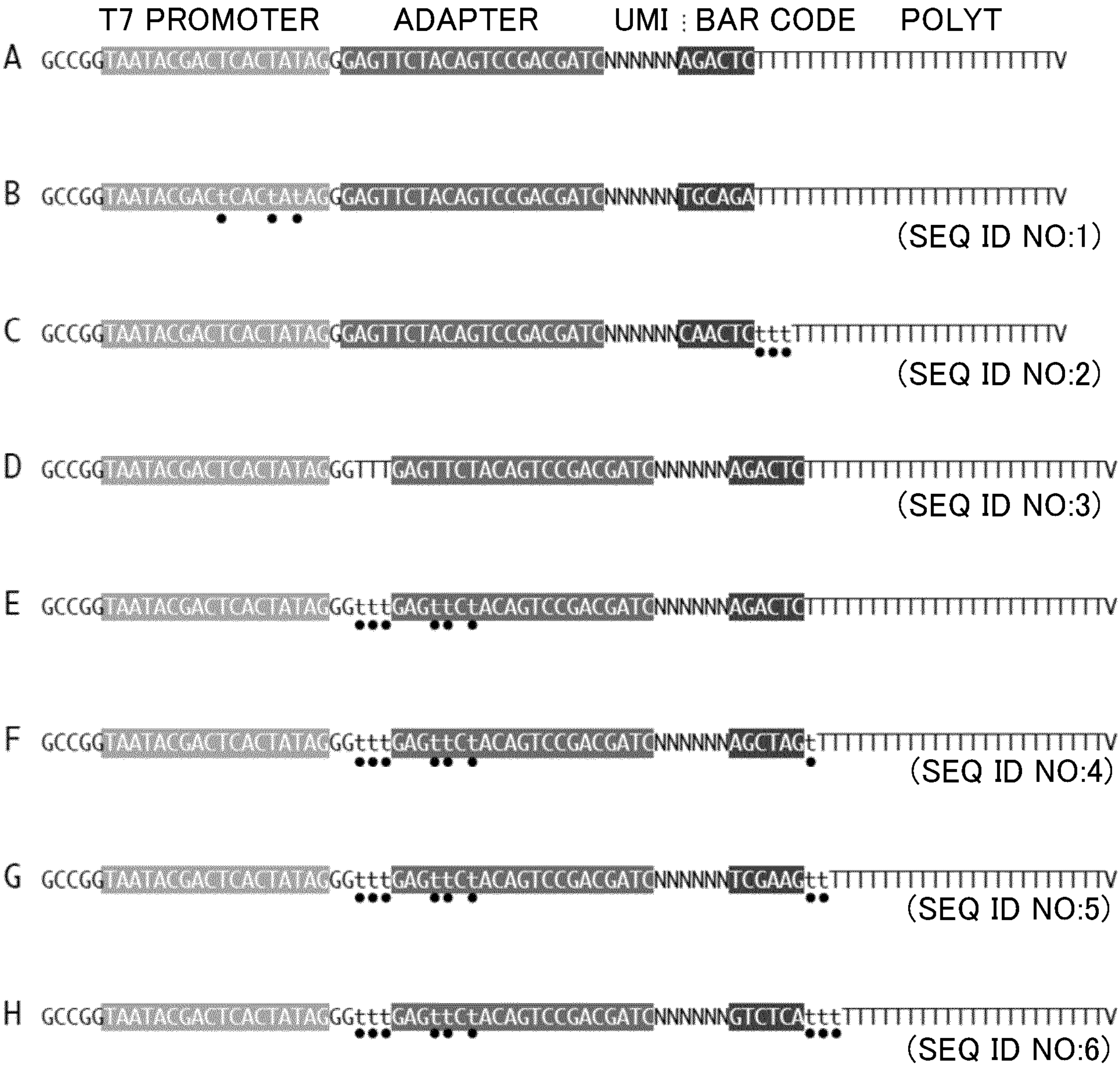
T7 PROMOTER
ADAPTER
UMI
BAR CODE
POLYT
A
B
(SEQ ID NO:1)
C
(SEQ ID NO:2)
D
(SEQ ID NO:3)
E
F
(SEQ ID NO:4)
G
(SEQ ID NO:5)
H
(SEQ ID NO:6)

FIG. 5

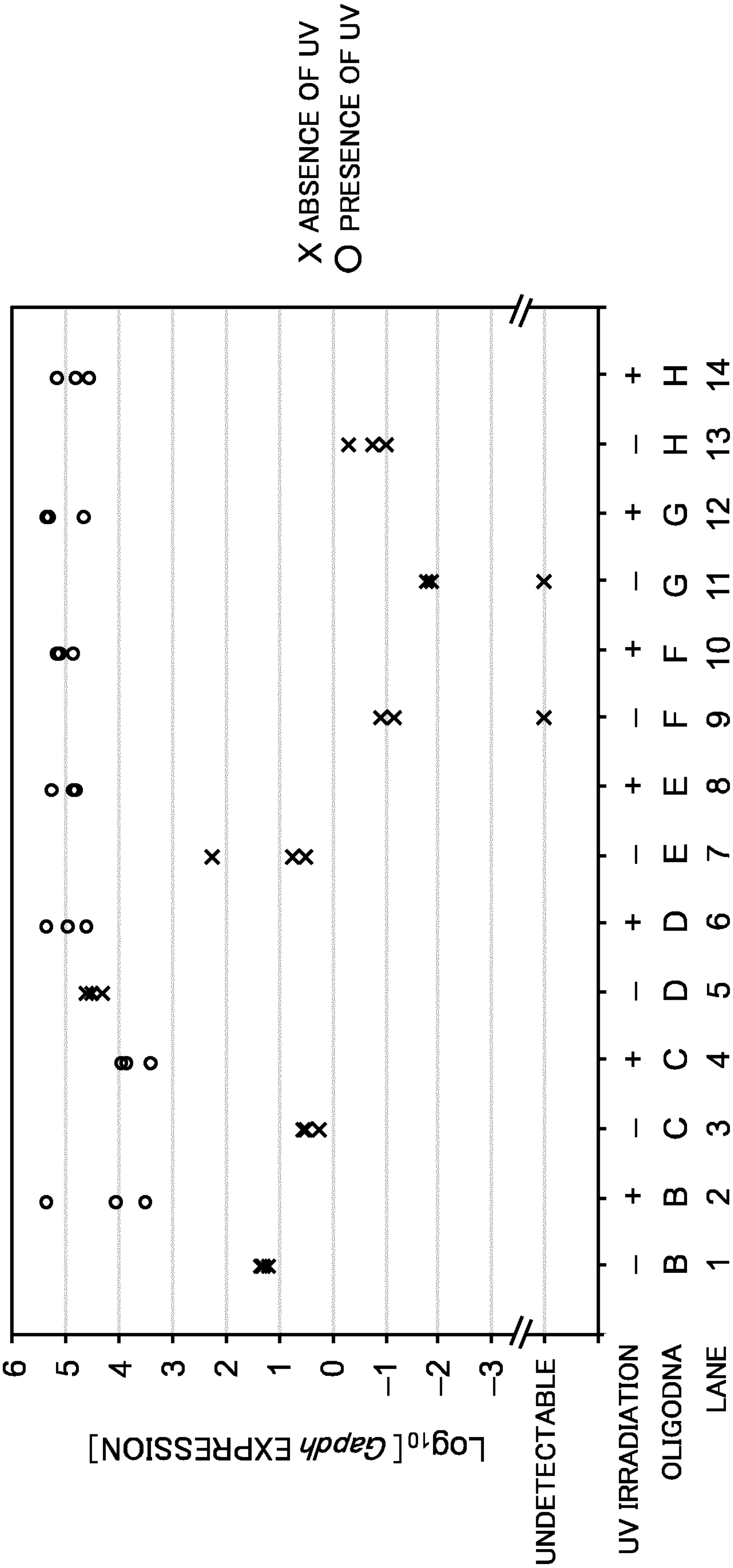
X ABSENCE OF UV
O PRESENCE OF UV
Log10[ Gapdh EXPRESSION]
6
5
4
3
2
1
0
−1
−2
−3
UNDETECTABLE
UV IRRADIATION − + − + − + − + − + − + − +
OLIGODNA B B C C D D E E F F G G H H
LANE 1 2 3 4 5 6 7 8 9 10 11 12 13 14

FIG. 6

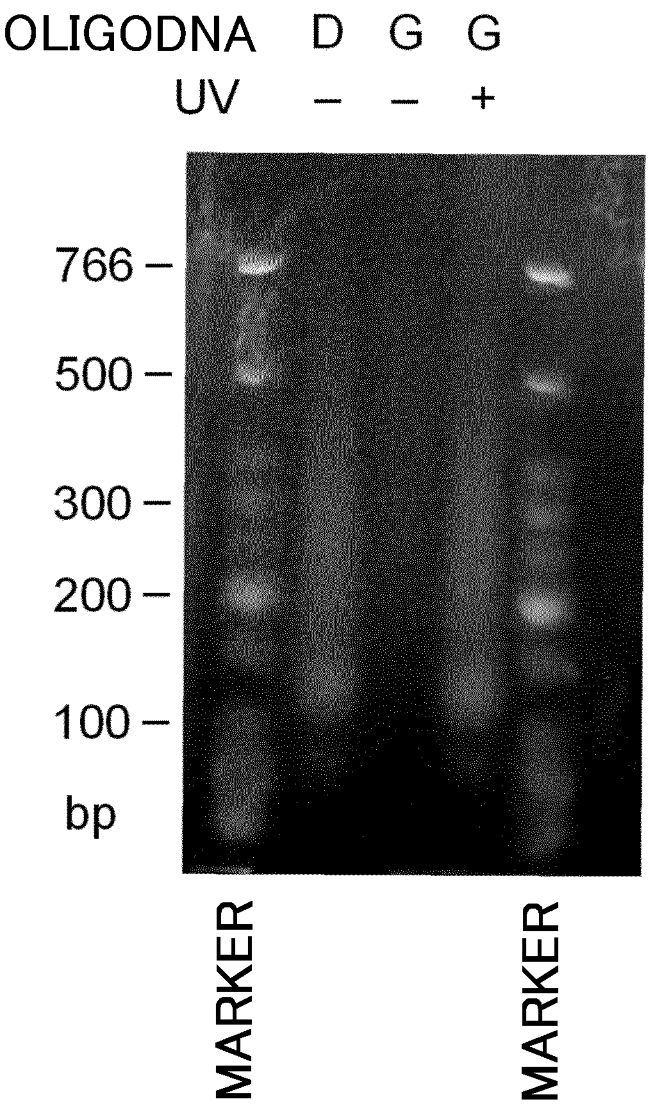

FIG. 7A

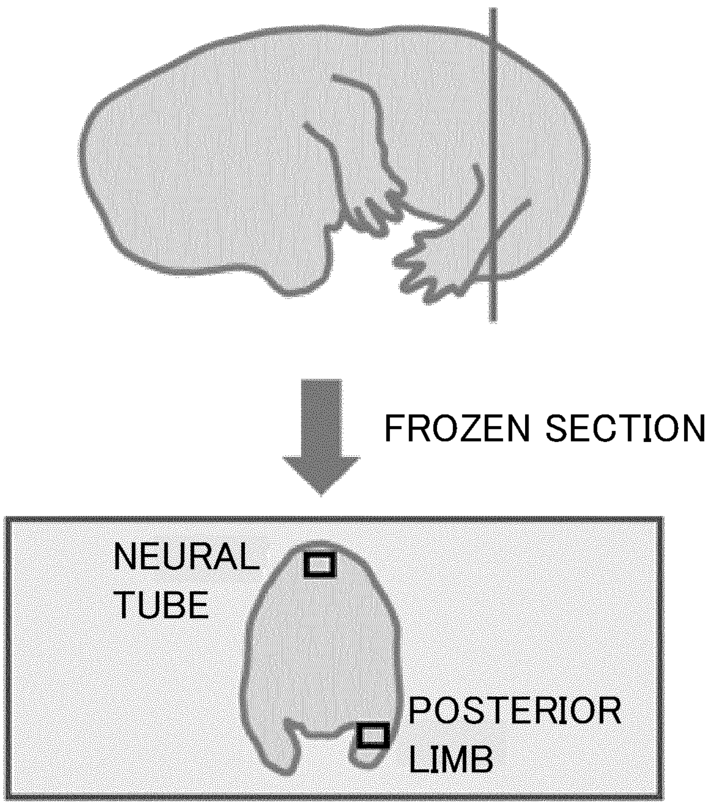
FROZEN SECTION
NEURAL TUBE
POSTERIOR LIMB

FIG. 7B

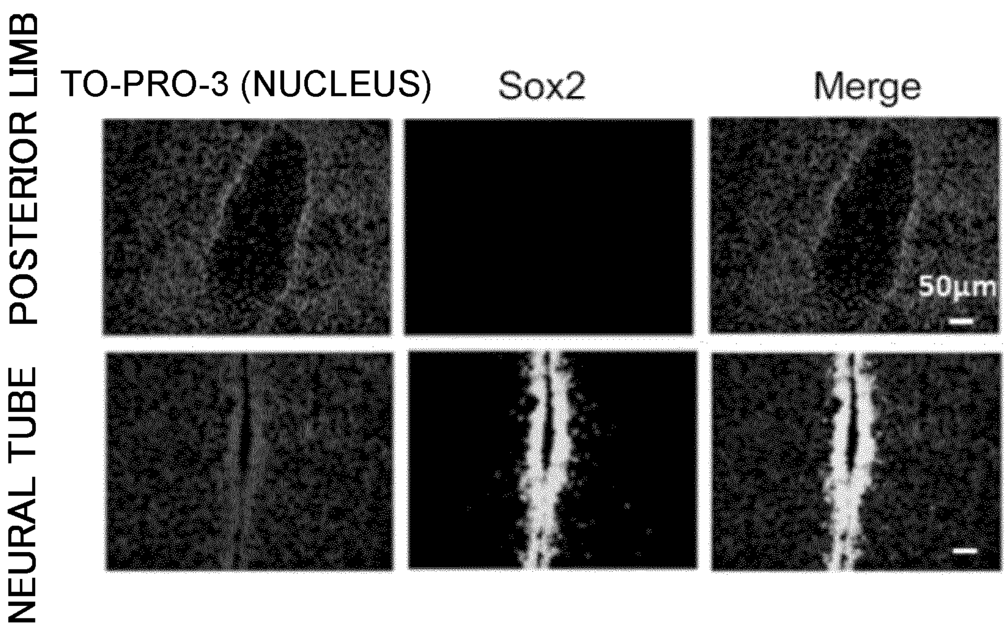
POSTERIOR LIMB
NEURAL TUBE
TO-PRO-3 (NUCLEUS)
Sox2
Merge
50μm

FIG. 8B

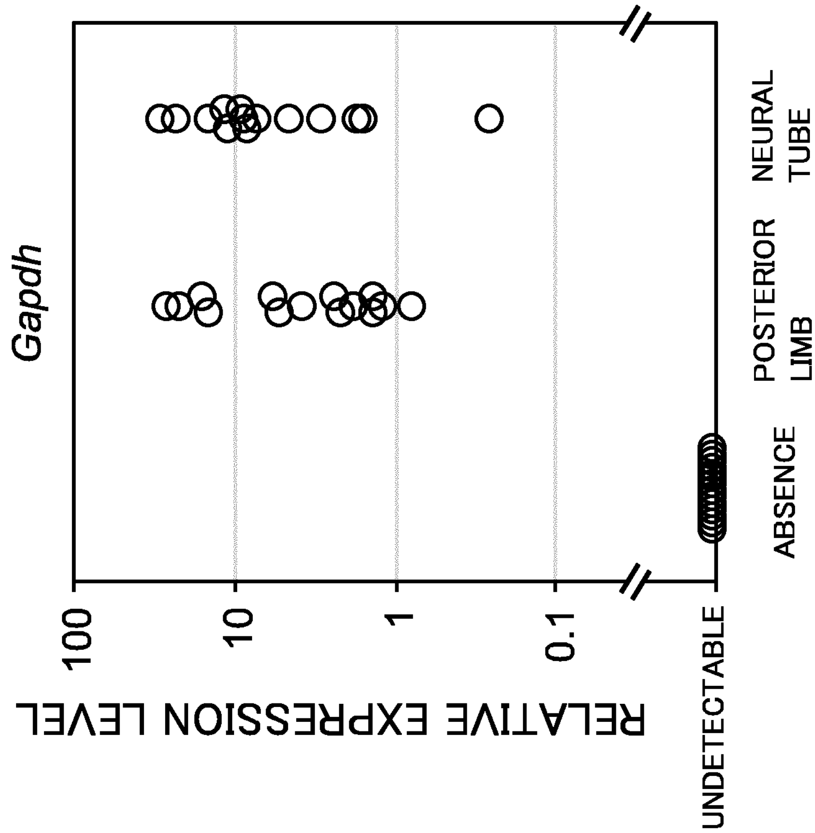

Gapdh
RELATIVE EXPRESSION LEVEL
100
10
1
0.1
UNDETECTABLE
ABSENCE
POSTERIOR LIMB
NEURAL TUBE


FIG. 8A

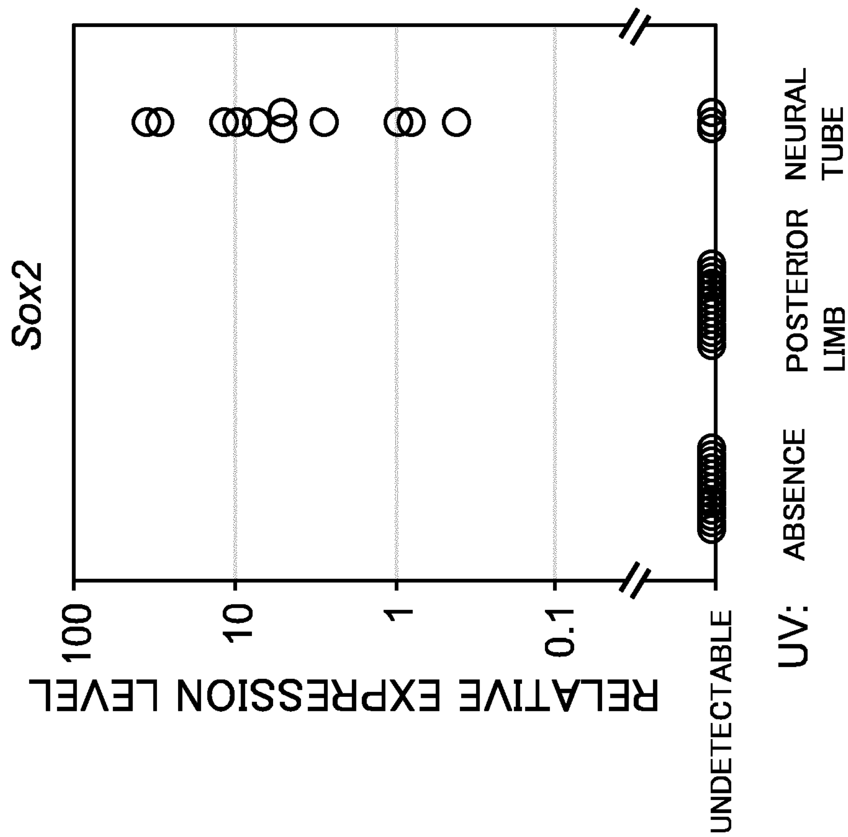

Sox2
RELATIVE EXPRESSION LEVEL
100
10
1
0.1
UNDETECTABLE
UV:
ABSENCE
POSTERIOR LIMB
NEURAL TUBE

FIG. 9

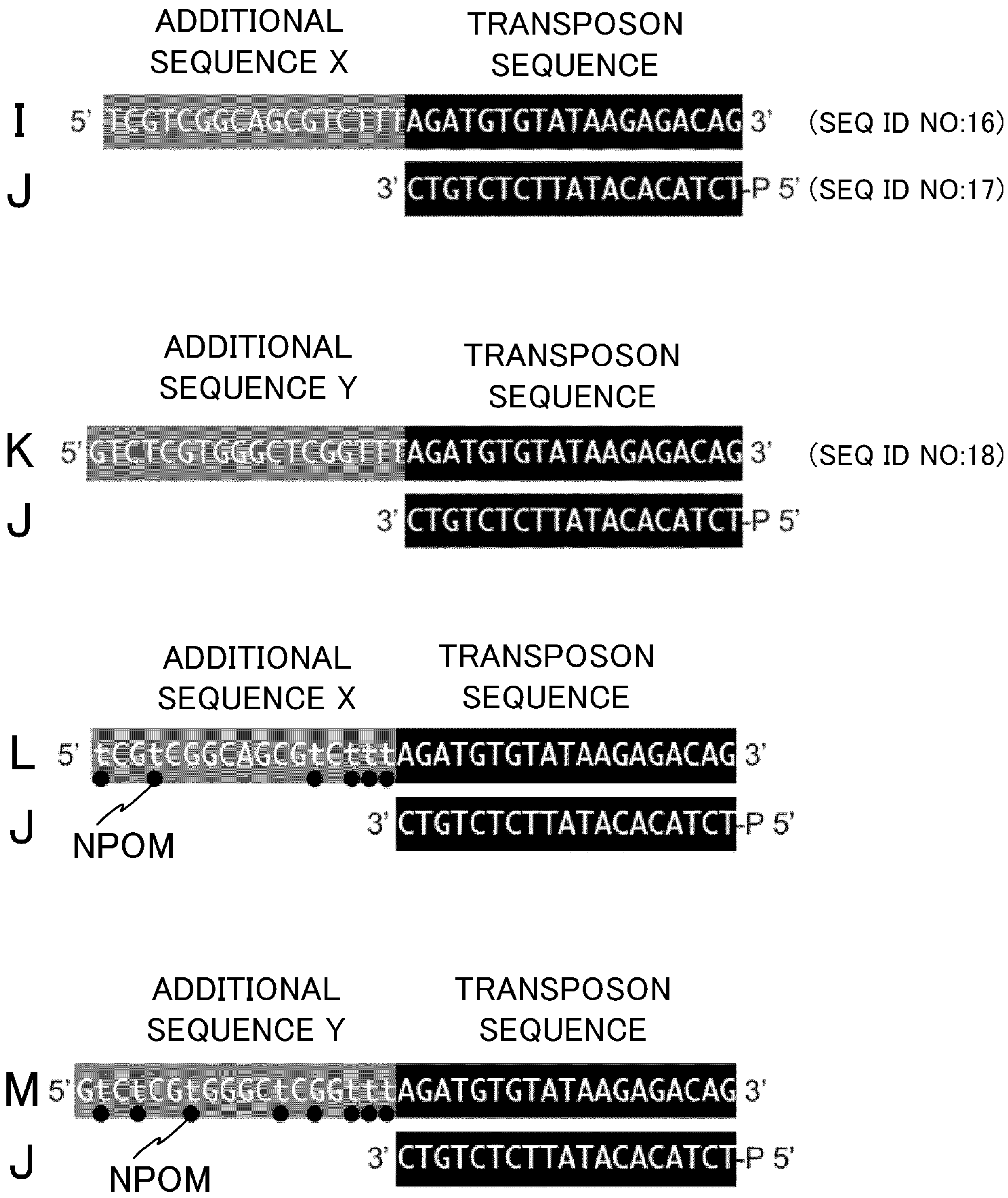
ADDITIONAL SEQUENCE X
TRANSPOSON SEQUENCE
I 5' TCGTCGGCAGCGTCTTTAGATGTGTATAAGAGACAG 3' (SEQ ID NO:16)
J 3' CTGTCTCTTATACACATCT-P 5' (SEQ ID NO:17)
ADDITIONAL SEQUENCE Y
TRANSPOSON SEQUENCE
K 5' GTCTCGTGGGCTCGGTTTAGATGTGTATAAGAGACAG 3' (SEQ ID NO:18)
J 3' CTGTCTCTTATACACATCT-P 5'
ADDITIONAL SEQUENCE X
TRANSPOSON SEQUENCE
L 5' tCGtCGGCAGCGtCtttAGATGTGTATAAGAGACAG 3'
J 3' CTGTCTCTTATACACATCT-P 5'
NPOM
ADDITIONAL SEQUENCE Y
TRANSPOSON SEQUENCE
M 5' GtCtCGtGGGCtCGGtttAGATGTGTATAAGAGACAG 3'
J 3' CTGTCTCTTATACACATCT-P 5'
NPOM

# FIG. 10

| LANE NUMBER | 1 | 2 | 3 | 4 | 5 | 6 | 7 | 8 |
|---|---|---|---|---|---|---|---|---|
| FIXATION | − | − | + | + | + | + | + | + |
| PROTECTING GROUP | − | − | − | − | + | + | + | + |
| UV | − | − | − | − | − | − | + | + |
| THE NUMBER OF CYCLES | 31 | 31 | 25 | 25 | 31 | 31 | 31 | 31 |

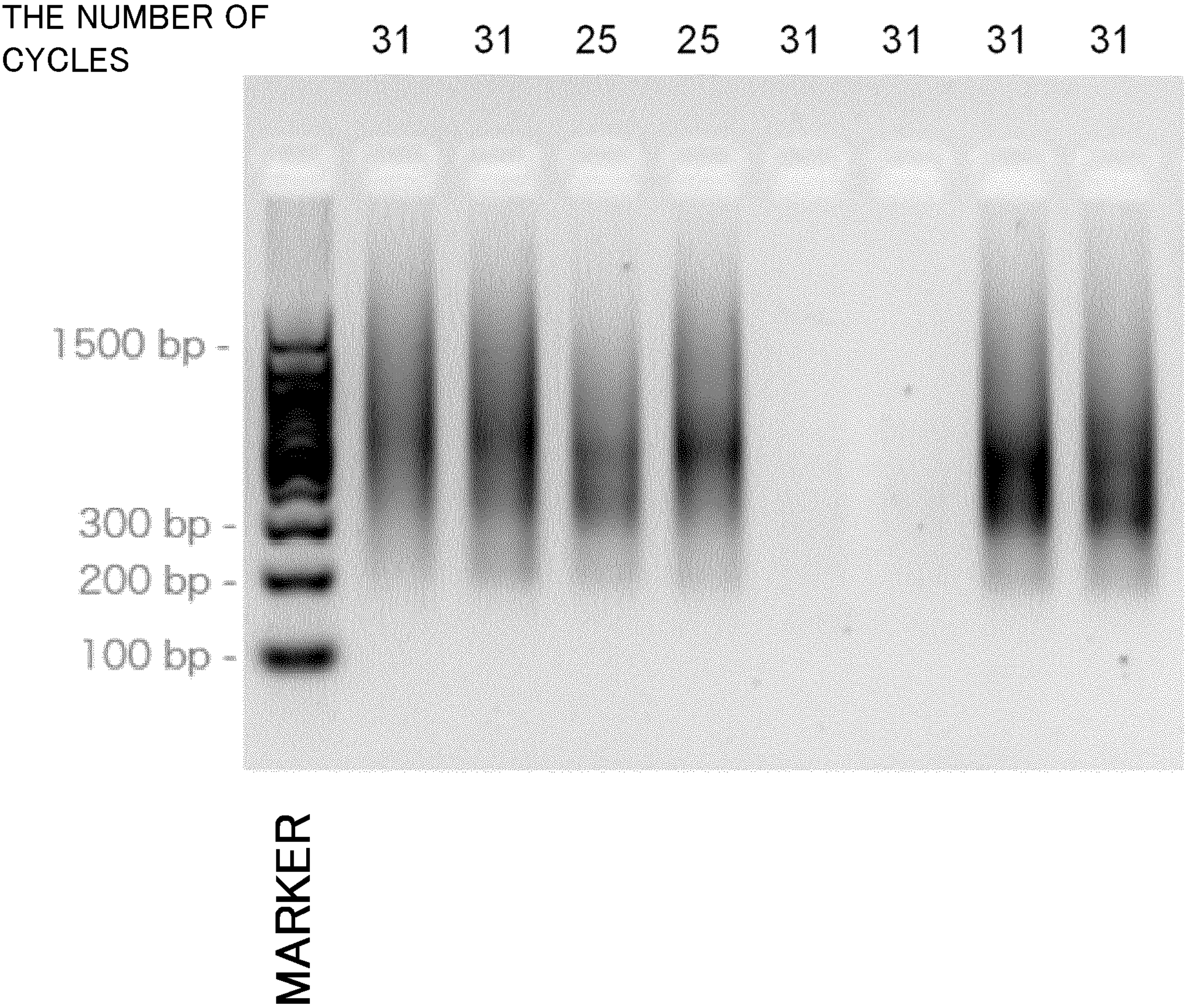

## INTERNATIONAL SEARCH REPORT

International application No.
PCT/JP2020/019790

A. CLASSIFICATION OF SUBJECT MATTER
C12N 15/11(2006.01)i; C12Q 1/68(2018.01)i
FI: C12N15/11 ZNA; C12Q1/68
According to International Patent Classification (IPC) or to both national classification and IPC

B. FIELDS SEARCHED
Minimum documentation searched (classification system followed by classification symbols)
C12N15/11; C12Q1/68

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>A | US 2017/0233722 A1 (UNIVERSITY OF WASHINGTON) 17.08.2017 (2017-08-17) paragraphs [0033], [0072], [0068], example 3, fig. 16, 17 | 1, 2, 6, 9, 10<br>3-5, 7, 8 |
| X<br>A | JP 2017-507656 A (VENTANA MEDICAL SYSTEMS, INC.) 23.03.2017 (2017-03-23) claims 51, 52, 62, 64, paragraphs [0024], [0046], [0109], [0114], examples 9, 10, fig. 6(A)-(D), 22, SEQ ID NO: 38-41, 47, 48 | 1-4, 7, 8, 10<br>5, 6, 9 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 July 2020 (28.07.2020) | 11 August 2020 (11.08.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/019790

| Patent Documents referred in the Report | Publication Date | Patent Family | Publication Date |
|---|---|---|---|
| US 2017/0233722 A1 | 17 Aug. 2017 | (Family: none) | |
| JP 2017-507656 A | 23 Mar. 2017 | US 2016/0362730 A1 claims 51, 52, 62, 64, paragraphs [0046], [0068], [0131], [0136], examples 9, 10, fig. 6(A)-(D), fig. 22, SEQ ID NO: 38-41, 47, 48<br>WO 2015/128272 A2<br>EP 3126512 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019094216 A

### Non-patent literature cited in the description

- **TAMAR HASHIMSHONY.** CEL-Seq: Single-Cell RNA-Seq by Multiplexed Linear Amplification. *Cell Rep.,* 2012, vol. 2 (3), 666-673
- **TAMAR HASHIMSHONY.** CEL-Seq2: sensitive highly-multiplexed single-cell RNA-Seq. *Genome Biology,* 2016, vol. 17 (77